(19) Europäisches Patentamt
European Patent Office
Office européen des brevets

(11) Veröffentlichungsnummer: **0 405 255 A2**

# (12) EUROPÄISCHE PATENTANMELDUNG

(21) Anmeldenummer: **90111337.3**

(51) Int. Cl.5: **C07D 223/22**, A61K 31/55

(22) Anmeldetag: **15.06.90**

(30) Priorität: **30.06.89 DD 330175**

(43) Veröffentlichungstag der Anmeldung:
**02.01.91 Patentblatt 91/01**

(84) Benannte Vertragsstaaten:
**AT CH DE ES FR GB IT LI SE**

(71) Anmelder: **VEB Arzneimittelwerk Dresden**
**Wilhelm-Pieck-Strasse 35 Postfach 89/90**
**DDR-8122 Radebeul 1(DD)**

(72) Erfinder: **Wunderlich, Helmut, Dr.**
**Westendring 22**
**DDR-8027 Dresden(DD)**
Erfinder: **Stark, Andreas, Dr.**
**Robert-Koch-Strasse 12**
**DDR-8122 Radebeul(DD)**
Erfinder: **Lohmann, Dieter, Dr.**
**Hoflössnitzstrasse 30**
**DDR-8122 Radebeul(DD)**
Erfinder: **Zenker, Lothar, Dr.**
**Sachsenstrasse 27**
**DDR-8122 Radebeul(DD)**
Erfinder: **Bartsch, Reni, Dr.**
**Stephensonstrasse 11**
**DDR-8045 Dresden(DD)**
Erfinder: **Poppe, Hildegard, Dr.**
**Kieler Strasse 6**
**DDR-8080 Dresden(DD)**
Erfinder: **Skoldinov, Aleksandr Petrovic, Prof. Dr.**
**Casovaja Strasse 25, Korpus 2, Wohnung 99**
**Moskau 125315(SU)**
Erfinder: **Kaverina, Natalja Veniaminovna, Prof. Dr.**
**W. Ulbricht Strasse 3, Wohnung 32**
**Moskau 125057(SU)**
Erfinder: **Grizenko, Anna Nikiticna, Dr.**
**Jenisseijskaja Strasse 28, Korpus 2, Wohnung 213**
**Moskau 129281(SU)**
Erfinder: **Lyskovzev, Valentin Viktorovic, Dr.**
**M. Molcanovka Strasse 6, Wohnung 1**
**Moskau G-69(SU)**
Erfinder: **Grigoreva, Ekaterina Klementevna, Dr.**
**Sirokaja Strasse 19, Korpus 2, Wohnung 193**
**Moskau 129224(SU)**

(74) Vertreter: **Patentanwälte Beetz sen. - Beetz jun. Timpe - Siegfried - Schmitt-Fumian-Mayr**
**Steinsdorfstrasse 10**
**D-8000 München 22(DE)**

## (54) 3-Carbalkoxyamino-5-aminoacyl-5H-dibenz[b,f]-azepine, ihre Salze und Verfahren zu ihrer Herstellung.

(57) Die Erfindung betrifft 3-Carbalkoxyamino-5-(aminoacyl)-5H-dibenz [b,f] azepine der Formel I,

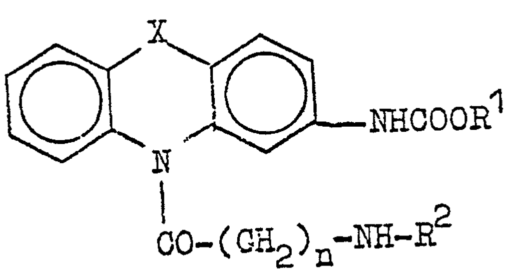

(I),

EP 0 405 255 A2

worin bedeuten:
$R^1$ geradkettiges oder verzweigtes $C_{1-3}$-Alkyl,
$R^2$ Wasserstoff, $C_{1-6}$-Alkyl, der geradkettig, verzweigt oder ringgeschlossen sein kann, Aralkyl oder β-Oxethyl
n 1, 2, 3, 4 oder 5
und
X $-CH_2-CH_2-$ oder $-CH=CH-$,
sowie ihre Salze, insbesondere die physiologisch verträglichen Salze,
wobei die Verbindung der Formel I mit $R^1$ = Ethyl, $R^2$ = Methyl, n = 1 und X = $-CH_2-CH_2-$ ausgenommen ist, ein Verfahren zu ihrer Herstellung sowie pharmazeutische Mittel mit den Verbindungen der Formel I als Wirkstoffen.

Das Verfahren beruht auf der Umsetzung eines 3-Carbalkoxyamino-5-halogenacyl-5H-dibenz [b,f] azepins mit einem Amin zum gewünschten Zielprodukt, das gegebenenfalls in ein physiologisch verträgliches Salz überführt wird.

Die Verbindungen der Formel I sind aufgrund ihrer ausgeprägten antiarrhythmischen Wirkung in der Therapie von Herzrhythmusstörungen verwendbar.

# NEUE 3-CARBALKOXYAMINO-5-AMINOACYL-5H-DIBENZ[B,F] AZEPINE SOWIE IHRE HERSTELLUNG UND PHARMAZEUTISCHE VERWENDUNG

Die Erfindung betrifft neue 3-Carbalkoxyamino-5-aminoacyl-derivate des 5H-Dibenz [b,f] azepins der allgemeinen Formel I,

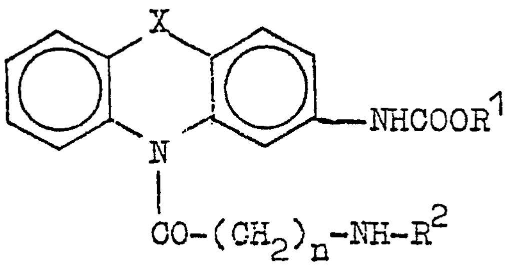

(I),

worin bedeuten:

$R^1$ einen geradkettigen oder verzweigten Alkylrest mit 1 bis 3 C-Atomen,

$R^2$ Wasserstoff oder einen Alkylrest mit 1 bis 6 C-Atomen, der geradkettig, verzweigt oder ringgeschlossen sein kann, oder einen Aralkylrest, wie Benzyl- oder Phenylethyl, oder einen $\beta$-Oxethylrest,

n eine ganze Zahl von 1 bis 5

und

X $-CH_2-CH_2-$ oder $-CH=CH-$,

ihre Salze mit anorganischen oder organischen Säuren, insbesondere physiologisch verträglichen Säuren, mit Ausnahme der Verbindung der Formel I mit $R^1$ = Ethyl, $R^2$ = Methyl, n = 1 und X = $-CH_2-CH_2-$, Verfahren zu ihrer Herstellung sowie pharmazeutische Mittel mit diesen Verbindungen als Wirkstoffen.

Die Verbindungen besitzen wertvolle antiarrhythmische Wirkungen und können zur Therapie von Herzrhythmusstörungen (Arrhythmien) vorteilhaft eingesetzt werden.

Die Verbindungen der allgemeinen Formel I sind sowohl bezüglich ihrer Herstellung als auch bezüglich ihrer antiarrhythmischen Wirkungen nicht vorbeschrieben. Lediglich die Verbindung 3-Carbethoxyamino-5-methylaminoacetyl-10,11-dihydro-5H-dibenz [b,f] azepin ist bezüglich ihrer chemischen Herstellung (Die Pharmazie 40 (1985), 825-835), jedoch nicht bezüglich ihrer pharmakologischen Wirkung vorbeschrieben.

Von strukturverwandten, beschriebenen 3-Carbalkoxyamino-5dialkylaminoacyl-10,11-dihydro-5H-dibenz [b,f] azepinen, unter denen 3-Carbethoxyamino-5-dimethylaminoacetyl-10,11-dihydro-5H-dibenz [b,f] azepin gegenwärtig klinisch geprüft wird (DD-A-152 782; SU-A-1 089 089), unterscheiden sich die neuen Verbindungen grundsätzlich in der Amino gruppe, die bei den bekannten Verbindungen eine tertiäre Aminogruppe ist, während die erfindungsgemäßen Strukturen primäre oder sekundäre Amine darstellen.

Es ist offenkundig, daß dieser entscheidende strukturelle Unterschied nicht nur andere chemische Verfahrensweisen bei der Herstellung derartiger Verbindungen voraussetzt, sondern daß damit auch neue Gesichtspunkte bezüglich der pharmakologischen Wirkungen verbunden sind. Besonders gilt dies für das Verhalten solcher Verbindungen im Verlauf des biologischen Abbaus im Organismus, was von bemerkenswertem Einfluß auf die Resorption, Distribution, Biotransformation und Eliminierung und damit auf den Wirkungscharakter und die Wirkungsdauer ist.

Schließlich sind zahlreiche weitere Antiarrhythmika bekannt, die in der Therapie von Herzrhythmusstörungen mehr oder weniger breit angewendet werden. Hierzu rechnen u.a. Chinidin, Lidocain, Mexiletin, Verapamil, Propafenon, Disopyramid, Moracizin und Etacizin. Die teilweise stark unterschiedlichen chemischen Strukturen dieser Stoffe lassen keine allgemeingültigen Regeln zur Beschreibung von Struktur-Wirkungs-Beziehungen erkennen. Insbesondere zeigen die genannten Therapeutika keine offenkundigen Strukturzusammenhänge mit den erfindungsgemäßen Verbindungen der allgemeinen Formel I.

Durch die Erfindung werden neue, hocheffektive Herz-Kreislauf-Pharmaka bereitgestellt, die aufgrund ihrer ausgeprägten antiarrhythmischen Wirkung in der Therapie von Herzrhythmusstörungen eingesetzt werden können. Mit den erfindungsgemäßen Strukturen wird nicht nur eine hohe Wirksamkeit und Spezifität der Wirkung erreicht, sondern es werden auch Nachteile herkömmlicher Wirkstoffe im Hin blick auf die Biotransformation vermieden, was sich günstig auf die Wirkungsdauer auswirkt.

Neben der hohen Wirksamkeit trägt auch die gute Verträglichkeit der erfindungsgemäßen Verbindungen, insbesondere das Fehlen von ungünstigen psychotropen Effekten, wie Sedierung und zentrale Erregung, dazu bei, daß die Therapiesicherheit bei der Behandlung von Herzrhythmusstörungen verbessert wird.

Die Erfindung hat die Aufgabe, neue, hocheffektive antiarrhythmisch wirksame 3-Carbalkoxyamino-5H-dibenz [b,f] azepine anzugeben, die auf technisch einfache Weise und unter Verwendung an sich bekannter Vor- und Zwischenprodukte herstellbar sind.

Die Aufgabe wird anspruchsgemäß gelöst. Die abhängigen Ansprüche betreffen vorteilhafte Ausführungsformen.

Das erfindungsgemäße Verfahren zur Herstellung der 3-Carbalkoxyamino-5-(aminoacyl)-5H-dibenz [b,f] azepine der allgemeinen Formel I und ihrer Salze ist gekennzeichnet durch

(A) Umsetzung eines 3-Carbalkoxyamino-5-halogenacyl-5H-dibenz [b,f] azepins der allgemeinen Formel II,

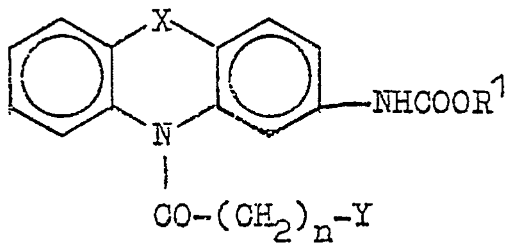

(II),

worin $R^1$, n und X dasselbe wie oben und
Y Chlor oder Brom bedeuten,
mit einem Amin der allgemeinen Formel III

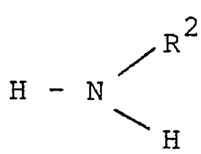

(III)

mit $R^2$ wie oben
zur entsprechenden Verbindung der Formel I
sowie gegebenenfalls

(B) Überführung der erhaltenen Verbindungen der Formel I mit anorganischen oder organischen Säuren in entsprechende Salze

oder gegebenenfalls Überführung von Salzen von Verbindungen der allgemeinen Formel I in die entsprechenden freien Basen.

Die zur Umsetzung erforderlichen Ausgangsstoffe der allgemeinen Formel II sind bekannt. Nach DD-A-152 782 und SU-A1 089 089 können sie aus 3-Carbalkoxyamino-5H-dibenz [b,f]azepinen durch Umsetzung mit einem Halogenacylhalogenid erhalten werden.

Die weiterhin zur Umsetzung erforderlichen Amine der allgemeinen Formel III sind technisch leicht zugängliche Ausgangsstoffe, wie z.B. Ammoniak, Methylamin, Ethylamin, n-Propylamin, $\beta$-Oxethylamin und n-Hexylamin sowie auch Isopropylamin, t-Butylamin, Cyclohexylamin, Benzylamin und Phenylethylamin. Die Amine können sowohl in reiner Form als auch in Form ihrer Lösungen in organischen Lösungs mitteln oder auch in wäßriger Lösung zur Anwendung kommen.

Die Umsetzung der Reaktionspartner zu den erfindungsgemässen Verbindungen der allgemeinen Formel I wird zweckmäßigerweise in Gegenwart von Lösungsmitteln durchgeführt. Als solche kommen vorzugsweise niedere Alkohole, wie Methanol, Ethanol oder n-Propanol, oder indifferente aliphatische oder aromatische Kohlenwasserstoffe, wie Cyclohexan, Benzol und Toluol, oder auch halogenierte Kohlenwasserstoffe, wie Chloroform, Tetrachlorkohlenstoff oder Chlorbenzol, zur Anwendung.

Die zur Reaktion notwendigen Amine der allgemeinen Formel III können sowohl in reiner Form, z.B. als gasförmiges Ammoniak, oder auch in ihren Lösungen in den oben genannten Lösungsmitteln für die Umsetzung verwendet werden. Es ist auch möglich - und kann in verschiedenen Fällen von Vorteil sein -, diese Amine als wäßrige Lösungen einzusetzen. Dies gilt vor allem für Ammoniak, Methylamin und Ethylamin.

Die erfindungsgemäße Umsetzung kann sowohl bei Raumtemperatur als auch bei erhöhten Temperaturen bis zur Siedetemperatur des Lösungsmittels oder Lösungsmittelgemischs erfolgen.

Da sich bei der erfindungsgemäßen Umsetzung von Verbindungen der allgemeinen Formel II mit Aminen der allgemeinen Formel III Halogenwasserstoff bildet, ist es für den Verlauf der Reaktion von Nutzen, wenn man zusätzlich Halogenwasserstoff-Acceptoren verwendet. Dazu eignen sich übliche anorganische Verbindungen, wie Natriumcarbonat oder Kaliumcarbonat, oder auch tertiäre Amine, wie Triethylamin oder Pyridin. Von besonderem Vorteil ist es, wenn man zur Halogenwasserstoff-Bindung das für die jeweilige erfindungsgemäße Reaktion erforderliche Amin der allgemeinen Formel III in einem entsprechenden Überschuß einsetzt. Zur Unterdrückung einer Nebenreaktion, die darin besteht, daß sich bei der erfindungsgemäßen Umsetzung der Halogenacylderivate der allgemeinen Formel II mit einem Amin der allgemeinen Formel III eine Verbindung der allgemeinen Formel IV bildet,

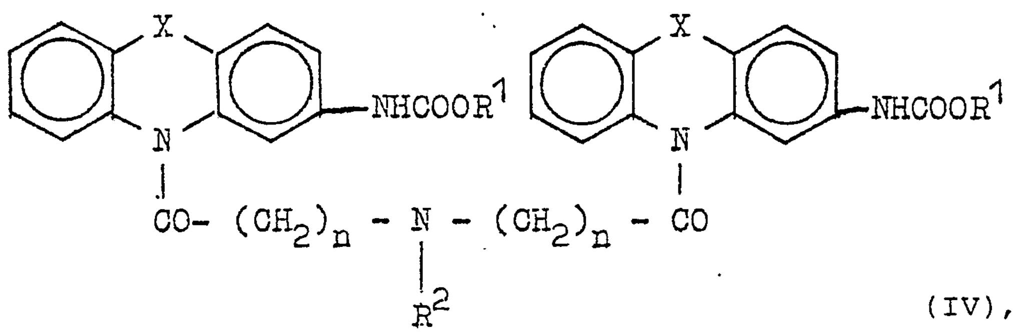

(IV),

worin X, n, $R^1$ und $R^2$ die gleiche Bedeutung besitzen wie oben,
ist es zweckmäßig, das jeweils verwendete Amin der allgemeinen Formel III in einer größeren Menge einzusetzen, als dies für die erfindungsgemäße Reaktion und für die Halogenwasserstoff-Bindung nach den Regeln der Stöchiometrie erforderlich ist. Im allgemeinen erzielt man einen günstigen Reaktionsverlauf, wenn man den Überschuß des Amins der allgemeinen Formel III bis zu 800 Stoffmengenanteile in % wählt, wobei die höheren Überschüsse vor allem dann zur Anwendung kommen, wenn es sich bei den Reaktionspartnern um leicht flüchtige Amine, wie Ammoniak oder Methylamin handelt. Es ist auch möglich, ohne Halogenwasserstoff-Acceptoren zu arbeiten. In einem solchen Fall kann die entstehende Verbindung der allgemeinen Formel I selbst als Halogenwasserstoff-Acceptor fungieren, so daß die Verbindungen der allgemeinen Formel I unmittelbar in Form ihrer entsprechenden Hydrohalogenide erhalten werden.

Die erfindungsgemäßen Reaktionsprodukte der allgemeinen Formel I stellen ölige oder kristalline Stoffe dar, die bei Gegenwart von Wasser oder Luftfeuchtigkeit als Hydrate vorliegen können. Die Verbindungen sind löslich in üblichen organischen Lösungsmitteln und schwer löslich in Wasser. Für die medizinische Anwendung eignen sich die erfindungsgemäßen Verbindungen insbesondere in Form ihrer wasserlöslichen Salze mit physiologisch verträglichen anorganischen oder organischen Säuren, wie Salzsäure, Schwefelsäure, Weinsäure, Citronensäure und anderen. Diese Salze können in an sich bekannter Weise hergestellt werden und können gegebenenfalls auch hydratisiert sein.

Aus solchen Salzen können durch Einwirkung von basisch reagierenden Stoffen, wie Alkalicarbonaten, Alkalihydroxiden, Aminen oder Ammoniak, in an sich bekannter Weise die erfindungsgemäßen Basen der allgemeinen Formel I freigesetzt werden, wobei man diesen Prozeß vorzugsweise im Verlauf der Reinigungsoperationen anwendet.

Die erfindungsgemäßen 5H-Dibenz [b,f] azepine der allgemeinen Formel I zeichnen sich bei der tierexperimentellen Untersuchung durch eine ausgeprägte antiarrhythmische Wirkung aus. In verschiedenen pharmakologischen Modellen zur Prüfung der antiarrhythmischen Wirksamkeit erwiesen sich die erfindungsgemäßen Verbindungen als wesentlich stärker wirksam als zum Vergleich herangezogene, in die Therapie von Herzrhythmusstörungen eingeführte Therapeutika, wie Lidocain, Mexiletin und Moracizin. Insbesondere zeigen die erfindungsgemäßen Verbindungen auch gegenüber den in DD-A-152 782 und SU-A-1 089 089 beschriebenen strukturverwandten 10,11-Dihydro-5H-dibenz [b,f] azepinen vorteilhafte Effekte, von denen sich das 3-Carbethoxyamino-5-dimethylaminoacetyl-10,11-dihydro-5H-dibenz [b,f] azepin in der klinischen Prüfung befindet.

In der nachstehenden Tabelle sind für typische erfindungsgemäße Verbindungen pharmakologische Daten aufgeführt.

Da sich bei etwa gleicher Verträglichkeit mit der erhöhten Wirkungsstärke eine am Aconitin-Modell der Ratte im Einzelfall sogar wesentliche Verbesserung der therapeutischen Breite Q ergibt (siehe die Tabelle,

Q = $LD_{50\ i.v.}/ED_{73\ i.v.}$), bieten sich die erfindungsgemäßen Verbindungen mit Vorzug für eine medizinische Verwendung bei der Behandlung von Herzrhythmusstörungen an.

Hinsichtlich ihrer Verträglichkeit ist besonders hervorzuheben, daß trotz einer den Psychopharmaka verwandten tricyclischen Grundstruktur weder sedierende noch zentral erregende oder andere psychisch ungünstige Wirkungen nachweisbar sind.

Hervorzuheben ist weiterhin, daß die erfindungsgemäßen Verbindungen der allgemeinen Formel I in vergleichenden Untersuchungen an der zweistufigen Koronarligatur am Hund (Harris-Modell) Vorteile bezüglich der Wirkungsdauer zeigen (siehe die Tabelle).

Für die Anwendung der erfindungsgemäßen Verbindungen in der Therapie von Herzrhythmusstörungen eignen sich verschiedenartige galenische Formulierungen, wie Tabletten, Dragees, Tropfenlösungen oder Injektionslösungen und andere Zübereitungsformen. In Abhängigkeit vom Schweregrad der Herzrhythmusstörung kann die einmal oder mehrmals täglich oral zu applizierende Dosis in einem mittleren Dosisbereich von 5 bis 100 mg liegen.

| Nr. | n | x | $R^1$ | $R^2$ | Aconitin, Ratte $ED_{73}$ i.v. (mg/kg) | akute Tox. Ratte $LD_{50}$ i.v. (mg/kg) | Q | Harris-Modell, Hund eff. Dosis (mg/kg i.v.) | Harris-Modell, Hund Wirkungsdauer (min) |
|---|---|---|---|---|---|---|---|---|---|
| 1 | 1 | $-CH_2-CH_2-$ | $-C_2H_5$ | $-CH_2-$ 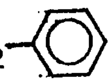 | 0,79 (0,53-1,2) | 13 (12 - 13) | 16 | - | - |
| 2 | 1 | $-CH_2-CH_2-$ | $-C_2H_5$ | $-CH_2-CH_2-OH$ | 0,19 (0,12-0,29) | 12 (11 - 13) | 63 | 2,0 | 60 |
| 3 | 1 | $-CH_2-CH_2-$ | $-C_2H_5$ | $-C_2H_5$ | 0,09 (0,048-0,17 | 9,6 (8,6 - 11) | 107 | 1,0<br>2,0 | 60<br>180 |
| 4 | 1 | $-CH_2-CH_2-$ | $-C_2H_5$ | $i-C_3H_7$ (iso) | 0,71 (0,40-1,3) | 8,2 (7,8-8,7) | 12 | - | - |
| 5 | 1 | $-CH_2-CH_2-$ | $-C_2H_5$ | $-n-C_3H_7$ | 0,18 (0,12-0,28) | 6,1 (5,5-6,8) | 34 | - | - |
| 6 | 1 | $-CH_2-CH_2-$ | $-C_2H_5$ | $-t-C_4H_9$ | 0,35 (0,23-0,54) | 5,6 (5,2-6,0) | 16 | - | - |
| 7 | 1 | $-CH_2-CH_2-$ | $-C_2H_5$ | $-n-C_4H_9$ | 0,29 (0,19-0,45) | 5,0 (4,0-6,3) | 17 | - | - |
| 8 | 1 | $-CH_2-CH_2-$ | $-C_2H_5$ | Cyclohexyl | 0,25 (0,18-0,33) | 3,7 (3,2-4,4) | 15 | - | - |

Fortsetzung der Tabelle

| | | | | | | | | | |
|---|---|---|---|---|---|---|---|---|---|
| 9 | 1 | $-CH_2-CH_2-$ | $-C_2H_5$ | $-n-C_6H_{13}$ | 0,90 (0,48-1,7) | 14 (13 - 16) | 16 | - | - |
| 10 | 1 | $-CH_2-CH_2-$ | $-C_2H_5$ | $-H$ | 0,20 (0,13-0,32) | 14 (13 - 15) | 70 | - | - |
| 11 | 1 | $-CH_2-CH_2-$ | $-i-C_3H_7$ | $-CH_3$ | 0,61 (0,40-0,93) | 15 (15 - 16) | 25 | - | - |
| 12 | 1 | $-CH_2-CH_2-$ | $-i-C_3H_7$ | $-CH_2-CH_2-OH$ | 0,51 (0,33-0,80) | 20 (19 - 22) | 39 | - | - |
| 13 | 1 | $-CH_2-CH_2-$ | $-i-C_3H_7$ | $-C_2H_5$ | 0,47 (0,24-0,9) | 16 (14 - 17) | 34 | - | - |
| 14 | 1 | $-CH_2-CH_2-$ | $-i-C_3H_7$ | $-t-C_4H_9$ | 0,18 (0,14-0,23) | - | - | - | - |
| 15 | 1 | $-CH_2-CH_2-$ | $-CH_3$ | $-CH_3$ | 0,30 (0,075-1,2) | 20 (19 - 22) | 67 | 2,0 | 15 |
| 16 | 1 | $-CH_2-CH_2-$ | $-CH_3$ | $-CH_2-CH_2-OH$ | 0,40 (0,29-0,55) | 36 (28 - 45) | 90 | - | - |
| 17 | 1 | $-CH_2-CH_2-$ | $-CH_3$ | $-C_2H_5$ | 0,95 (0,35-2,6) | 19 (16 - 21) | 20 | 2,0 | 15 |
| 18 | 2 | $-CH_2-CH_2-$ | $-C_2H_5$ | $-CH_2-CH_2-OH$ | 0,85 (0,50-1,5) | 20 (17 - 20) | 24 | 2,0 | 60 |

Fortsetzung der Tabelle

| | | | | | | | | | |
|---|---|---|---|---|---|---|---|---|---|
| 19 | 1 | $-CH=CH-$ | $-C_2H_5$ | $-CH_3$ | 0,32 (0,20-0,49) | 11 (9,6 - 12) | 34 | – | – |
| 20 | 1 | $-CH=CH-$ | $-C_2H_5$ | $-C_2H_5$ | 0,40 (0,25-0,64) | 6,2 (5,8 - 6,5) | 15 | – | – |
| 21 | 2 | $-CH_2-CH_2-$ | $-C_2H_5$ | $-C_2H_5$ | 0,42 (0,24-0,75) | 12 (11 - 13) | 29 | – | – |
| 22 | 1 | $-CH_2-CH_2-$ | $-C_2H_5$ | 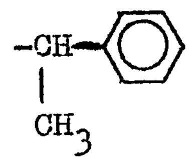 | 3,5 (2,1-5,8) | 31 (29 - 34) | 9 | – | – |
| | | Lidocain | | | 9,2 (5,9-15) | 18 (17 - 21) | 2,0 | 6,0 | 10 |
| | | Moracizin | | | 1,1 (0,41-3,1) | 11 (9,6 - 12) | 10 | 2,0 | 15 |
| | | Bonnecor(R) | | | 0,28 (0,13-0,62) | 11 (10 - 12) | 39 | 2,0 | 15 |
| | | Mexiletin | | | 15 (8,8-25) | 41 (34 - 50) | 2,7 | – | – |

## Legende zur Tabelle

$ED_{73}$ i.v. = Dosis in mg/kg, welche die Aconitinarrhythmie an weiblichen Wistarratten nach i.v.-Applikation der Testsubstanz bis zum Auftreten vereinzelter ventrikulärer Extrasystolen hemmt (Femmer, K., und Mitarb., Pharmazie 31 , S. 36, 1976), Berechnung nach der Probit-Regressionsanalyse mit Vertrauensbereich p = 0,05 (Werte in Klammern)

$LD_{50}$ iv. = Letale Dosis in mg/kg bei 50 % der Versuchstiere einer orientierenden akuten Toxizität an männlichen oder weiblichen Wistarratten hauseigener Zucht, i.v.-Applikation in die Schwanzvene, 3 - 4 Dosierungsgruppen zu je 10 Tieren. Berechnung nach der Probit-Regressionsanalyse mit Vertrauensbereich p = 0,05 (Werte in Klammern)

eff. Dosis = effektive Dosis in mg/kg sowie Wirkungsdauer in Minuten an Hunden, 24 h nach zweistufiger Koronarligatur (Kaverina and Mitarb., Pharmazie 12 , S. 845, 1985)

$$Q = \text{Quotient} \quad \frac{LD_{50}\ \text{i.v.}}{ED_{73}\ \text{i.v.}} \quad \text{(therapeutische Breite).}$$

## Ausführungsbeispiele

**Beispiel 1:** 5-Benzylaminoacetyl-3-carbethoxyamino-10,11-dihydro-5H-dibenz[b,f]azepin

18 g (0,05 mol) 3-Carbethoxyamino-5-chloracetyl-10,11-dihydro-5H-dibenz [b,f] azepin (A) werden in einem 500-ml-Dreihalskolben unter Rühren in 120 ml Ethanol suspendiert und nach Zugabe von 20 g Benzylamin (ca. 0,2 mol) 4 bis 5 h im Wasserbad zum Sieden erhitzt. Nach ca. 1 h liegt eine fast klare Lösung vor. Unter der Reaktion scheidet sich kein Benzylamin-HCl ab. Das Reaktionsgemisch wird über Nacht unter Kühlung stehengelassen; es tritt sehr gute Kristallbildung ein. Nach Absaugung wird der Filterkuchen mit Ethanol und Wasser nachgewaschen (Entfernung von Benzylamin-HCl).
Fp.: 155 - 157 °C.

Die Base ist in verdünnten Säuren schwer löslich.

| Elementaranalyse: | | | |
|---|---|---|---|
| Molekülmasse M: 429,52 | | | |
| | C (%) | H (%) | N (%) |
| berechnet: | 72,70 | 6,34 | 9,78 |
| gefunden : | 72,63<br>72,63 | 6,53<br>6,54 | 9,75<br>9,79 |

**Beispiel 2:** 3-Carbethoxyamino-5-(β-oxethylamino)-acetyl-10,11-dihydro-5H-dibenz [b,f] azepin

36 g (0,1 mol) 3-Carbethoxyamino-5-chloracetyl-10,11-dihydro-5H-dibenz [b,f] azepin (A) werden in 200 ml Ethanol suspendiert, mit 19 g Ethanolamin (0,32 mol) versetzt und 4 h am Rückfluß gekocht. Nach Heißfiltration wird die Lösung zur Trockne eingeengt und der verbleibende zähflüssige Rückstand in 100 ml

Aceton aufgenommen. Die klare, farblose acetonische Lösung wird mit isopropanolischer Salzsäure bis pH 2 - 3 versetzt und im Kühlschrank zur Kristallisation gebracht. Das Kristallisat wird abgesaugt, mit wenig Aceton nachgewaschen und getrocknet.
Ausbeute: 25 g = 59,5 % der Theorie.
Rohprodukt-Fp.: 204 - 208 °C.
Umkristallisation aus Gemisch Isopropanol/Ethanol unter Zusatz von Aktivkohle.
Umkristallisat-Ausbeute: 15 g
Fp.: 219 - 224 °C.

| Elementaranalyse: | | | | |
|---|---|---|---|---|
| | C (%) | H (%) | N (%) | Cl (%) |
| berechnet: | 60,06 | 6,24 | 10,01 | 8,44 |
| gefunden : | 59,79<br>59,84 | 6,33<br>6,27 | 9,80<br>9,82 | 8,41<br>8,55 |

**Beispiel 3:** 3-Carbethoxyamino-5-monoethylaminoacetyl-10,11-dihydro-5H-dibenz [b,f] azepin

72 g (0,2 mol) A werden in 1000 ml Ethanol (96 %ig) suspendiert und unter Rühren mit 150 g 30 %iger wäßriger Monoethylaminlösung (1 mol) versetzt. Anschließend wird zunächst 1 h bei 60 °C und danach 4 h bei 70 - 75 °C gerührt. Die leicht abgekühlte Gesamtmischung wird in 2500 ml Wasser/Eis-Mischung eingegossen und nach längerem Stehen zur Kristallisation gebracht. Das Kristallisat wird über eine Filternutsche abgesaugt und mit Wasser aminfrei gewaschen.
Ausbeute: 71 g = 96,6 % der Theorie.
Rohprodukt-Fp.: 152-159 °C.
Umkristallisation aus Aceton (1 g aus 10 ml)
Fp.: 157 - 160 °C.
Summenformel: $C_{21}H_{25}N_3O_3$
Molekülmasse: 367,45

| Elementaranalyse: | | | |
|---|---|---|---|
| | C (%) | H (%) | N (%) |
| berechnet: | 68,64 | 6,86 | 11,44 |
| gefunden: | 68,63<br>68,67 | 6,93<br>7,01 | 11,22<br>11,26 |

**Beispiel 4:** 3-Carbethoxyamino-5-isopropylaminoacetyl-10,11-dihydro-5H-dibenz [b,f] azepin

36 g (0,1 mol) A werden in 250 ml Ethanol (96 %ig) suspendiert und nach Zugabe von 18 g (0,3 mol) Isopropylamin 5 h am Rückfluß gekocht. Nach Heißfiltration wird zur Trockne eingeengt und der zähflüssige Rückstand in 250 ml Chloroform aufgenommen und zweimal mit 100 ml Wasser ausgeschüttelt. Die Chloroformphase wird erneut zur Trockne eingeengt und der blasige Rückstand in 400 ml Aceton kalt gelöst.
Nach längerem Stehen kristallisiert eine schwach gelbe Substanz. Ausbeute 10,5 g = 25 % der Theorie.
Fp.: 224-228 °C (nach Umkristallisation aus Ethanol).

| Elementaranalyse: Hydrochlorid | | | | |
|---|---|---|---|---|
| | C (%) | H (%) | N (%) | Cl (%) |
| berechnet: | 63,22 | 6,75 | 10,05 | 8,48 |
| gefunden : | 63,18<br>63,24 | 6,86<br>6,96 | 9,99<br>10,20 | 8,28<br>8,38 |

**Beispiel 5:** 3-Carbethoxyamino-5-n-propylaminoacetyl-10,11-dihydro-5H-dibenz [b,f] azepin

36 g (0,1 mol) A werden in 250 ml Ethanol (96 %ig) suspendiert und nach Zugabe von 20 g (ca. 0,33 mol) n-Propylamin 4 bis 5 h am Rückfluß gekocht. Anschließend werden 80 bis 100 ml Ethanol aus der Reaktionsmischung abdestilliert; die heiß filtrierte Restlösung wird nach längerem Stehen zur Kristallisation gebracht. Die Substanz wird abgesaugt und mit wenig Alkohol nachgewaschen.
Ausbeute: 25 g = 65,5 % der Theorie.
Fp.: 156-160 °C.
Summenformel: $C_{22}H_{27}N_3O_3$
Molekülmasse: 381,48

| Elementaranalyse: Base | | | |
|---|---|---|---|
| | C (%) | H (%) | N (%) |
| berechnet: | 69,27 | 7,13 | 11,01 |
| gefunden : | 69,18<br>69,04 | 7,15<br>7,15 | 10,81<br>10,78 |

**Beispiel 6:** 5-t-Butylaminoacetyl-3-carbethoxyamino-10,11-dihydro-5H-dibenz [b,f] azepin

36 g (0,1 mol) A werden in 250 ml Ethanol vorgelegt und mit 22 g (0,3 mol) t-Butylamin versetzt. Die Mischung wird 5 h am Rückfluß gekocht und nach Abkühlung von ca. 8 g un-umgesetztem Ausgangsprodukt A abgesaugt. Das klare Filtrat wird mit der doppelten Menge Wasser versetzt und das ausgefällte Produkt abgesaugt (29 g). Nach Reinigung über eine Umfällung (aus 250 ml 1n HCl und 250 ml Ethanol nach Filtration mit $NH_3$ wieder als Base ausgefällt) resultieren 27 g Rohprodukt. Diese werden in 150 ml Ethanol und 70 ml 1n HCl gelöst und nach Filtration im Vakuum zur Trockne eingedampft. Der gesamte Rückstand wird mit 200 ml Aceton 30 min am Rückfluß gekocht, von Ungelöstem abgesaugt und das Filtrat zur Kristallisation stehengelassen. Es resultieren nach längerem Stehen 13 g (Ausbeute 28,9 %) farblose Kristalle. Fp.: 182-196 °C.

| Elementaranalyse: Hydrochlorid-Hydrat | | | | |
|---|---|---|---|---|
| | C (%) | H (%) | N (%) | Cl (%) |
| berechnet: | 61,39 | 7,16 | 9,34 | 7,88 |
| gefunden : | 61,46<br>61,53 | 7,20<br>7,17 | 9,61 | 7,82<br>8,01 |

**Beispiel 7:** 5-n-Butylaminoacetyl-3-carbethoxyamino-10,11-dihydro-5H-dibenz [b,f] azepin

36 g (0,1 mol) A werden mit 250 ml Ethanol und 30 g (0,4 mol) n-Butylamin versetzt; das Gemisch wird 4 h am Rückfluß gekocht. Anschließend werden ca. 50 ml Ethanol abdestilliert; nach Heißfiltration wird die Gesamtlösung in den Kühlschrank gegeben. Entstandenes Kristallisat wird abgesaugt und mit wenig Ethanol nachgewaschen und getrocknet.
Ausbeute: 33 g = 83,4 % der Theorie.
Fp.: 184-188 °C.

| Elementaranalyse: Base | | | |
|---|---|---|---|
| | C (%) | H (%) | N (%) |
| berechnet: | 69,85 | 7,39 | 10,62 |
| gefunden : | 69,53<br>69,75 | 7,50<br>7,55 | 10,40<br>10,70 |

**Beispiel 8:** 3-Carbethoxyamino-5-cyclohexylaminoacetyl-10,11-dihydro-5H-dibenz [b,f] azepin

36 g (0,1 mol) A werden in 250 ml Toluol mit 30 g (0,3 mol) Cyclohexylamin 4 h am Rückfluß gekocht und noch heiß von Cyclohexylamin-Hydrochlorid abgesaugt. Die Lösung wird mit Wasser aminfrei gewaschen und nach Trocknung über Natriumsulfat zur Trockne eingedampft. Der Rückstand wird in einer Mischung aus 100 ml 2n HCl und 50 ml Ethanol gelöst und zur Kristallisation gebracht. Das Kristallisat wird abgesaugt, mit der gleichen HCl/Ethanol-Mischung nachgewaschen und getrocknet.
Ausbeute: 38,5 g.
Rohprodukt-Fp.: 167-175 °C.
Die Gesamtmenge wird mit 400 ml Aceton ausgekocht.
Ausbeute: 27 g = 56,7 % der Theorie.
Fp.: 182-188 °C.

| Elementaranalyse: Hydrochlorid-Hydrat | | | | |
|---|---|---|---|---|
| | C (%) | H (%) | N (%) | Cl (%) |
| berechnet: | 63,08 | 7,20 | 8,83 | 7,45 |
| gefunden : | 61,78 | 7,23 | 8,75<br>8,83 | 7,57<br>7,45 |

**Beispiel 9:** 3-Carbethoxyamino-5-n-hexylaminoacetyl-10,11-dihydro-5H-dibenz [b,f] azepin

36 g (0,1 mol) A werden in 250 ml Ethanol mit 35 g (0,35 mol) n-Hexylamin 4 h am Rückfluß gekocht; anschließend wird zur Trockne eingedampft. Der Rückstand wird mit 200 ml $CHCl_3$ und 400 ml warmem Wasser ausgeschüttelt, die Chloroform-Phase nach Abtrennung nochmals mit Wasser nach-extrahiert und nach Trocknung über Natriumsulfat erneut zur Trockne eingedampft. Der Rückstand wird in 250 ml Isopropanol mit 2 g Aktivkohle gekocht, heiß abgesaugt und zur Kristallisation stehengelassen.
Ausbeute: 18,5 g = 43,7 % der Theorie.
Fp.: 121-123 °C (Base).

| Elementaranalyse: Base | | | |
|---|---|---|---|
| | C (%) | H (%) | N (%) |
| berechnet: | 70,89 | 7,85 | 9,92 |
| gefunden : | 70,71<br>70,91 | 7,80<br>7,90 | 9,98<br>9,91 |

**Beispiel 10:** 5-Aminoacetyl-3-carbethoxyamino-10,11-dihydro-5H-dibenz [b,f] azepin

1,75 g (0,005 mol) A werden mit 1,7 g (0,1 mol) Ammoniak in 50 ml Ethanol im Autoklaven 3 h bei 56-58 °C geschüttelt und danach weitere 4 h auf 140-150 °C erwärmt. Nach Abkühlung wird das Reaktionsgemisch im Vakuum eingedampft und der erhaltene Rückstand in ca. 120 ml 5 %iger HCl gelöst. Die saure Lösung wird unter Zusatz von wenig Aktivkohle geblankt und mit verdünnter Natronlauge alkalisiert; die erhaltene Base wird aus Toluol umkristallisiert.
Ausbeute: 1,12 g = 68 % der Theorie.
Fp.: 158-159 °C.

| Elementaranalyse: Base | | | |
|---|---|---|---|
| | C (%) | H (%) | N (%) |
| berechnet:<br>gefunden : | 67,24<br>67,62 | 6,24<br>6,38 | 12,38<br>12,51 |

**Beispiel 11:** 3-Carbisopropoxyamino-5-methylaminoacetyl-10,11-dihydro-5H-dibenz [b,f] azepin

18 g (ca. 0,05 mol) 3-Carbisopropoxyamino-5-chloracetyl-10,11-dihydro-5H-dibenz [b,f] azepin (B) werden mit 300 ml Ethanol (96 %ig) in einem 2-1-Dreihalskolben vorgelegt und unter Rühren bei Raumtemperatur mit 100 ml wäßriger Monomethylaminlösung (38 %ig), ≙ 1,2 mol) versetzt. Auf einem Wasserbad wird das Reaktionsgemisch langsam auf 50 °C aufgeheizt, 3 h bei dieser Temperatur gerührt und dann 1 h bei 60 °C nachreagieren gelassen. Nach Abkühlung auf 25-30 °C werden 750 ml kaltes Wasser zugegeben und nach Stehen über Nacht erneut 400 ml Wasser unter Rühren zugesetzt. Von dem gebildeten öligen Niederschlag wird das Wasser-Amin-Alkohol-Gemisch abdekantiert und die gleiche Menge frisches Wasser zugesetzt. Nach erneutem Dekantieren wird der ölige Rückstand mit 80 ml Aceton in der Wärme gelöst, über Filter geblankt und im Kühlschrank zur Kristallisation gebracht.
Ausbeute: 11 g = 60 % der Theorie.
Fp.: 150-152 °C.
Summenformel: $C_{21}H_{25}N_3O_3$.
Molekülmasse: 367,46

| Elementaranalyse: | | | |
|---|---|---|---|
| | C (%) | H (%) | N (%) |
| berechnet: | 68,62 | 6,86 | 11,38 |
| gefunden : | 68,66<br>68,74 | 6,91<br>6,98 | 11,25<br>11,39 |

**Beispiel 12:** 3-Carbisopropoxyamino-5-($\beta$-oxyethyl)-aminoacetyl-10,11-dihydro-5H-dibenz [b,f] azepin

3,7 g (0,01 mol) B werden in 60 ml Toluol mit 3 g (0,05 mol) Ethanolamin 4 h am Rückfluß gekocht. Nach Abkühlung wird mit Wasser ausgeschüttelt, das ausgefallene Öl abgetrennt und aus Ethylacetat umkristallisiert. Es resultieren 2,5 g ( = 57 % der Theorie) Base.
Fp.: 138-149 °C.

| Elementaranalyse: | |
|---|---|
| | N (%) |
| berechnet: | 10,57 |
| gefunden : | 10,81 |

Hydrochlorid aus Toluol + etherische HCl
Fp.: 160 °C (Zersetzung).

| Elementaranalyse: | | |
|---|---|---|
| | N (%) | Cl (%) |
| berechnet: | 9,68 | 8,16 |
| gefunden : | 9,30/9,34 | 7,80/7,90 |

**Beispiel 13:** 3-Carbisopropoxyamino-5-ethylaminoacetyl-10,11-dihydro-5H-dibenz [b,f] azepin

3,7 g (0,01 mol) B, 10 g (0,04 mol) wäßrige Ethylaminlösung (33 %ig) und 35 ml Toluol werden 5-7 h bei 50 °C gerührt. Nach Abkühlung und Waschen mit Wasser wird die Toluolphase eingedampft und der verbleibende Rückstand aus 20 ml Isopropanol umkristallisiert.
Ausbeute: 2,35 g = 61 % der Theorie.
Fp.: 152-153 °C.

| Elementaranalyse: | | |
|---|---|---|
| | C (%) | H (%) |
| berechnet: | 69,26 | 7,13 |
| gefunden : | 69,22/69,37 | 7,17/7,23 |

Hydrochlorid aus Toluol + etherische HCl
Fp.: 180-185 °C (Umkristallisation aus Isopropanol).

**Beispiel 14:** 5-t-Butylaminoacetyl-3-carbisopropoxyamino-10,11-dihydro-5H-dibenz [b,f] azepin

3,7 g (0,01 mol) B werden mit 3,6 g t-Butylamin (0,05 mol) in 30 ml Ethanol 4 h am Rückfluß gekocht. Nach Aufarbeitung resultieren 3 g Base ≙ 75 % der Theorie.
Fp.: 154-156 °C (aus Toluol).

| Elementaranalyse: | | | |
|---|---|---|---|
| | C (%) | H (%) | N (%) |
| berechnet: | 67,35 | 7,77 | 9,83 |
| gefunden : | 67,61 | 7,53 | 9,84 |

Hydrochlorid aus Isopropanol + etherische HCl
Fp.: 185-187 °C.

| Elementaranalyse: | | | |
|---|---|---|---|
| | C (%) | H (%) | Cl (%) |
| berechnet: | 62,12 | 7,16 | 7,64 |
| gefunden : | 62,42 | 7,22 | 7,21 |

**Beispiel 15:** 3-Carbomethoxyamino-5-methylaminoacetyl-10,11-dihydro-5H-dibenz [b,f] azepin

2,4 g (0,006 mol) 3-Carbomethoxyamino-5-chloracetyl-10,11-dihydro-5H-dibenz [b,f] azepin (C) werden mit 1,2 g (0,04 mol) Methylamin in 30 ml Toluol 3 h in einem Autoklaven bei 40-50 °C geschüttelt und anschließend 4 h auf 150-155 °C aufgeheizt. Nach Aufarbeitung und Fällung des Hydrochlorids in Toluol mit etherischer HCl resultieren 1,2 g ≙ 46 % der Theorie. Fp.: 254-256 °C.

| Elementaranalyse: | | | |
|---|---|---|---|
| | C (%) | H (%) | N (%) |
| berechnet: | 60,72 | 5,89 | 11,18 |
| gefunden : | 61,15 | 5,98 | 11,38 |

**Beispiel 16:** 3-Carbomethoxyamino-5-(β-oxethyl)-aminoacetyl-10,11-dihydro-5H-dibenz [b,f] azepin

2,4 g (0,006 mol) C werden mit 1,8 g (0,03 mol) Ethanolamin in 70 ml Toluol 4 h am Rückfluß gekocht. Nach Aufarbeitung resultiert eine ölige Base, die in Toluol mit etherischer HCl als Hydrochlorid gefällt wird. Fp.: 220 °C (aus Ethanol).

| Elementaranalyse: | | | |
|---|---|---|---|
| | C (%) | H (%) | Cl (%) |
| berechnet: | 57,93 | 6,14 | 9,00 |
| gefunden : | 57,57 | 6,04 | 9,16/9,26 |

**Beispiel 17:** 3-Carbomethoxyamino-5-ethylaminoacetyl-10,11-dihydro-5H-dibenz [b,f] azepin

7 g (0,02 mol) C werden in 120 ml Toluol suspendiert und mit 25 g (0,18 mol) wäßriger Ethylaminlösung (33 %ig) versetzt. Innerhalb 1 h wird auf 50 °C erwärmt und nach Zusatz von 30 ml Ethanol

(Löslichkeit) 4 h am Rückfluß gekocht. Nach Aufarbeitung wird der ölige Rückstand in Isopropanol gelöst und mit isopropanolischer HCl das Hydrochlorid gefällt.
Ausbeute: 5 g = 63 % der Theorie.
Fp.: 241-243 °C.

| Elementaranalyse: | | | |
|---|---|---|---|
| | C (%) | H (%) | Cl (%) |
| berechnet: | 61,61 | 6,20 | 9,09 |
| gefunden : | 61,30 | 6,38 | 9,09 |

**Beispiel 18:** 3-Carbethoxyamino-5-β-(β-oxethylamino)propionyl-10,11-dihydro-5H-dibenz [b,f] azepin

1,75 g (0,01 mol) 3-Carbethoxyamino-5-β-chlorpropionyl-10,11-dihydro-5H-dibenz [b,f] azepin, 3 g (0,05 mol) Ethanolamin und 70 ml Ethanol werden 4-5 h am Rückfluß gekocht. Nach Aufarbeitung erhält man 3,4 g feste Base, die in Toluol gelöst und mit etherischer HCl in das Hydrochlorid überführt wird.
Ausbeute: 2,6 g = 60 % der Theorie.
Fp.: 186 - 187 °C (aus Isopropanol).

| Elementaranalyse: | | |
|---|---|---|
| | N (%) | Cl (%) |
| berechnet: | 9,68 | 8,17 |
| gefunden : | 9,90 | 7,98 |

**Beispiel 19:** 3-Carbethoxyamino-5-methylaminoacetyl-5H-dibenz [b,f] azepin

12 g (0,033 mol) 3-Carbethoxyamino-5-chloracetyl-5H-dibenz [b,f] azepin B werden in 150 ml Ethanol suspendiert. Man setzt 50 ml ca. 35 %ige wäßrige Methylaminlösung (ca. 0,5 mol $CH_3NH_2$) zu und rührt die Mischung bei 50-60 °C 5 h, wobei sich eine klare hellgelbe Lösung bildet. Das Reaktionsgemisch wird im Vakuum auf die Hälfte eingeengt und danach mit reichlich Wasser versetzt. Die ölig ausgefallene Base wird mit Chloroform (2 x 50 ml) extrahiert, die organische Phase mit Wasser gewaschen, über Natriumsulfat getrocknet und im Vakuum eingedampft. Es verbleibt eine Kristallmasse, die bei 148-153 °C schmilzt.
Ausbeute: 7,2 g.

Die Base wird in Isopropanol gelöst und mit isopropanolischer HCl das Hydrochlorid gebildet. Das Produkt wird abgesaugt, mit wenig Aceton gewaschen und getrocknet.
Ausbeute: 6 g = 46 % der Theorie.
Fp.: 243-270 °C.

| Elementaranalyse: | | | | |
|---|---|---|---|---|
| | C (%) | H (%) | N (%) | Cl (%) |
| berechnet: | 61,22 | 5,78 | 10,71 | 9,04 |
| gefunden : | 61,03 | 6,16 | 10,90 | 9,02 |

**Beispiel 20:** 3-Carbethoxyamino-5-ethylaminoacetyl-5H-dibenz [b,f] azepin

12 g B werden in 150 ml Ethanol suspendiert. Man setzt 70 ml ca. 33 %ige wäßrige Ethylaminlösung zu und rührt die Mischung bei 50-60 °C. Es bildet sich eine klare hellgelbe Lösung. Das Reaktionsgemisch wird im Vakuum auf die Hälfte eingeengt und mit reichlich Wasser versetzt. Die ölig anfallende Base wird durch Dekantieren der Wasserphase gewonnen und im Vakuum von restlichem Wasser befreit. Der Basenrückstand wird unter leichtem Erwärmen in Isopropanol gelöst, von geringen Trübungen abfiltriert und mit isopropanolischer HCl sauer gestellt. Nach mehrtägigem Stehen im Kühlschrank scheidet sich das Hydrochlorid in kristalliner Form ab. Es wird abgesaugt, mit wenig Isopropanol gewaschen und getrocknet.
Ausbeute: 11 g = 83 % der Theorie.
Umkristallisation aus n-Propanol (1:15) unter Zusatz von Aktivkohle. Ausbeute: 80 %, farblos. Fp.: 234-240 °C.

| Elementaranalyse: | | | | |
|---|---|---|---|---|
| | C (%) | H (%) | N (%) | Cl (%) |
| berechnet:<br>gefunden : | 62,76<br>62,52 | 6,02<br>6,36 | 10,46<br>10,42 | 8,82<br>8,57 |

**Beispiel 21:** 3-Carbethoxyamino-5-(β-ethylaminopropionyl)-10,11-dihydro-5H-dibenz [b,f] azepin

37,3 g (0,1 mol) 3-Carbethoxyamino-5-(β-chlorpropionyl)-10,11-dihydro-5H-dibenz [b,f] azepin werden in 250 ml 96 %igem Ethanol suspendiert. Man setzt 150 ml wäßrige Ethylaminlösung zu und rührt 6 h bei 50-60 °C. Anschliessend wird unter vermindertem Druck zur Trockne eingedampft und der Rückstand in 300 ml 1n HCl gelöst. Man behandelt die Lösung bei Raumtemperatur mit Aktivkohle und scheidet durch Zugabe von konz. Ammoniaklösung die Base ab. Die überstehende Lösung wird abdekantiert, das zähe Produkt mit Wasser gewaschen und in Ethylether aufgenommen. Durch Zugabe von isopropanolischer HCl wird auf pH 2 gestellt. Es kristallisiert 3-Carbethoxyamino-5-(β-ethylaminopropionyl)-10,11-dihydro-5H-dibenz [b,f] azepin-Hydrochlorid.
Ausbeute: 36 g
Fp.: ca. 150 °C.

| Elementaranalyse: | | | | |
|---|---|---|---|---|
| | C (%) | H (%) | N (%) | Cl (%) |
| berechnet:<br>gefunden : | 60,61<br>60,69 | 9,94<br>9,94 | 9,64<br>9,73 | 8,13<br>8,00 |

**Beispiel 22:** 3-Carbethoxyamino-5-alpha-phenylethylaminoacetyl-10,11-dihydro-5H-dibenz [b,f] azepin

18 g (0,05 mol) A werden in 150 ml Ethanol (96 %ig) mit 6 g (ca. 0,06 mol) Triethylamin und 12 g alpha-Phenylethylamin (ca. 0,1 mol) versetzt und 6-8 h am Rückfluß gekocht. Die klare gelbe Reaktionslösung wird mit 700-800 ml Wasser versetzt, die ölige Abscheidung durch Dekantieren abgetrennt und in 150 ml Aceton aufgenommen. Die farblose Acetonlösung wird mit 10 ml 2n HCl deutlich sauer gestellt, über Faltenfilter geblankt und zur Kristallisation stehengelassen. Nach Absaugung resultieren 22 g ≙ 91,5 % rohes Hydrochlorid; Fp.: 144-152 °C.
Nach Umkristallisation (Ethanol - wäßrige HCl) erhält man 14 g Produkt; Fp.: 155-161 °C.

| Elementaranalyse: | | | | |
|---|---|---|---|---|
| Hydrochlorid-Hydrat | | | | |
| Summenformel: $C_{27}H_{32}N_3O_4Cl$<br>Molekülmasse: 498 | | | | |
| | C (%) | H (%) | N (%) | Cl (%) |
| berechnet: | 65,12 | 6,48 | 8,44 | 7,12 |
| gefunden : | 65,15<br>65,12 | 6,57<br>6,58 | 8,40<br>8,52 | 7,50<br>7,60 |

## Ansprüche

1. 3-Carbalkoxyamino-5-(aminoacyl)-5H-dibenz [b,f] azepine der allgemeinen Formel I,

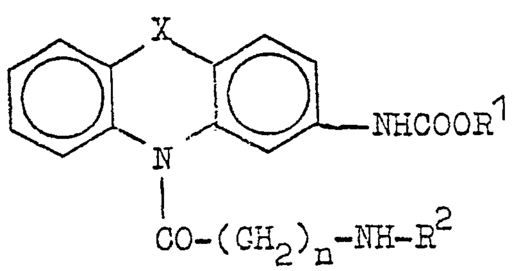

(I),

worin bedeuten:
$R^1$ einen geradkettigen oder verzweigten Alkylrest mit 1 bis 3 C-Atomen,
$R^2$ Wasserstoff, einen Alkylrest mit 1 bis 6 C-Atomen, der geradkettig, verzweigt oder ringgeschlossen sein kann, einen Aralkylrest, wie Benzyl oder Phenylethyl, oder einen $\beta$-Oxethylrest,
n 1, 2, 3, 4 oder 5
und
X $-CH_2-CH_2$ oder $-CH=CH-$,
sowie ihre Salze, insbesondere die physiologisch verträglichen Salze,
wobei die Verbindung der Formel I mit $R^1$ = Ethyl, $R^2$ = Methyl, n = 1 und X = $-CH_2-CH_2$ ausgenommen ist.

2. Verfahren zur Herstellung der 3-Carbalkoxyamino-5-(aminoacyl)-5H-dibenz [b,f] azepine der allgemeinen Formel I und ihrer Salze nach Anspruch 1,
gekennzeichnet durch
(A) Umsetzung eines 3-Carbalkoxyamino-5-halogenacyl-5H-dibenz [b,f] azepins der allgemeinen Formel II,

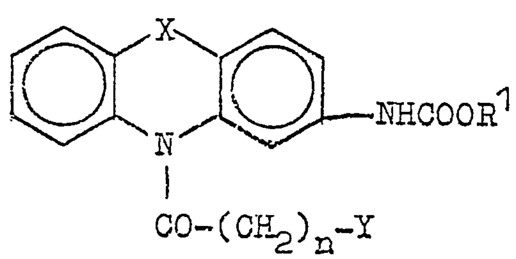

(II),

worin $R^1$, n und X dasselbe wie in Anspruch 1 und
Y Chlor oder Brom bedeuten,
mit einem Amin der allgemeinen Formel III

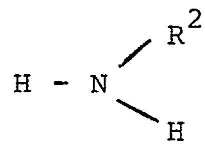

(III)

mit $R^2$ wie oben
zur entsprechenden Verbindung der Formel I
sowie gegebenenfalls
(B) Überführung der erhaltenen Verbindungen der Formel I mit anorganischen oder organischen Säuren in entsprechende Salze
oder gegebenenfalls Überführung von Salzen von Verbindungen der allgemeinen Formel I in die entsprechenden freien Basen.

3. Verfahren nach Anspruch 2, dadurch gekennzeichnet, daß Schritt A in organischen Lösungsmitteln, wie Benzol, Toluol, Chlorbenzol, Chloroform, Tetrachlorkohlenstoff, Methanol, Ethanol oder n-Propanol, gegebenenfalls in Gegenwart von Wasser, bei Temperaturen bis zur Siedetemperatur des Lösungsmittels oder des Lösungsmittelgemisches durchgeführt wird.

4. Verfahren nach den Ansprüchen 2 und 3, dadurch gekennzeichnet, daß Schritt A in Gegenwart von Halogenwasserstoff-Acceptoren, wie Natriumcarbonat oder Kaliumcarbonat oder tertiären Aminen, wie Triethylamin oder Pyridin, durchgeführt wird.

5. Verfahren nach Anspruch 4, dadurch gekennzeichnet, daß als Halogenwasserstoff-Acceptor das entsprechende Amin der allgemeinen Formel III in einem Überschuß von bis zu 800 Stoffmengenanteilen in % eingesetzt wird.

6. Verfahren nach den Ansprüchen 2 bis 5, dadurch gekennzeichnet, daß die erhaltene Verbindung der allgemeinen Formel I in an sich bekannter Weise mit einer physiologisch verträglichen anorganischen oder organischen Säure, wie Salzsäure, Schwefelsäure, Weinsäure, Citronensäure oder anderen, zum entsprechenden Salz umgesetzt wird, das gegebenenfalls hydratisiert ist.

7. Verfahren nach den Ansprüchen 2 bis 6, dadurch gekennzeichnet, daß aus einem Salz einer Verbindung der allgemeinen Formel I in an sich bekannter Weise, z.B. durch Freisetzung mit basisch reagierenden Stoffen, die entsprechende Verbindung der allgemeinen Formel I in Form ihrer freien Base hergestellt wird, die gegebenenfalls hydratisiert ist.

8. Verfahren nach Anspruch 7, dadurch gekennzeichnet, daß zur Freisetzung der Verbindung der allgemeinen Formel I in Form ihrer Base aus einem Salz Alkalicarbonate, Alkalihydroxide, Amine oder Ammoniak verwendet werden.

9. Pharmazeutische Mittel insbesondere zur Behandlung von Arrhythmien des Herz-Kreislauf-Systems, gekennzeichnet durch einen Gehalt an einer oder mehreren Verbindungen der Formel I nach Anspruch 1 und pharmazeutisch geeignete Hilfs- und/oder Trägerstoffen.